# EUROPÄISCHE PATENTSCHRIFT

(11) **EP 3 304 367 B1**
(45) Veröffentlichungstag und Bekanntmachung des Hinweises auf die Patenterteilung: **15.12.2021**
(21) Anmeldenummer: 16721644.9
(22) Anmeldetag: 06.04.2016
(51) Int. Cl.: G16H 40/63, G16H 30/20, G16H 30/40

(54) **VERFAHREN UND VORRICHTUNG ZUM ERZEUGEN EINER SERIE VON ABBILDUNGEN EINES OBJEKTS**
METHOD AND DEVICE FOR PRODUCING A SERIES OF IMAGES OF AN OBJECT
PROCÉDÉ ET DISPOSITIF POUR PRODUIRE UNE SÉRIE D'IMAGES D'UN OBJET

(30) Priorität: 27.05.2015 DE 102015108355
(43) Veröffentlichungstag der Anmeldung: 11.04.2018
(73) Patentinhaber: Fotofinder Systems GmbH, 84364 Bad Birnbach (DE)
(72) Erfinder: MAYER, Andreas, 94032 Passau (DE)
(74) Vertreter: advotec.
(86) Internationale Anmeldenummer: PCT/EP2016/057485
(87) Internationale Veröffentlichungsnummer: WO 2016/188659

(56) Entgegenhaltungen:
- EP-A1- 1 913 870
- WO-A2-2006/116700
- US-A1- 2011 090 348

## Beschreibung

Die Erfindung betrifft ein Verfahren zum Erzeugen einer Serie von Abbildungen eines Objekts sowie ein entsprechendes Abbildungssystem zum Erzeugen einer Serie von Abbildungen eines Objekts.

Derartige Systeme und Verfahren kommen beispielsweise im Umfeld medizinischer Behandlungen oder chirurgischer Eingriffe zum Einsatz. Die Verfahren und Vorrichtungen dienen dabei zur Kontrolle und/oder zur Dokumentation des Behandlungsfortschritts und/oder den Resultaten chirurgischer Eingriffe. Beispielsweise können mit gattungsgemäßen Vorrichtungen und Verfahren vor einem entsprechenden chirurgischen Eingriff eine Serie von Abbildungen erzeugt werden, die den zu behandelnden Körper in unterschiedlichen Haltungen und/oder räumlichen Ausrichtungen hinsichtlich einer Abbildungseinheit abbilden, wobei im Nachgang zu dem chirurgischen Eingriff eine oder mehrere entsprechende Serien von Abbildungen erzeugt werden, um die Heilung und/oder den Erfolg des chirurgischen Eingriffs zu dokumentieren.

Entsprechende Vorrichtungen und Verfahren können gleichermaßen auch bei der Dokumentation von Behandlungen eines Körpers zum Einsatz kommen, die keinen chirurgischen Eingriff erfordern. So können beispielsweise ästhetisch indizierte Behandlungen an einem Körper auch ohne chirurgischen Eingriff erfolgen, wobei gleichermaßen eine Dokumentation des Ausgangszustands des Körpers vor der Behandlung sowie die weitere Entwicklung des Körpers nach der Behandlung von besonderem Interesse ist. Als Beispiel sei hier die Entfernung von Tattoos mittels Laserstrahlung genannt.

Insgesamt soll es sich bei den abzubildenden Objekten im Sinne dieser Beschreibung um menschliche oder tierische Körper handeln. Zudem erfolgt die Erzeugung von Serien von Abbildungen der menschlichen oder tierischen Körper, wie nachfolgend offenbart, zur Dokumentation von medizinischen oder ästhetischen Eingriffen und/oder Behandlungen der menschlichen oder tierischen Körper.

Die so erzeugten Serien von Abbildungen bzw. der Vergleich von Abbildungen unterschiedlicher Bildserien und die aus dem Vergleich resultierende Aussagekraft hinsichtlich des Behandlungserfolges hängt zu einem hohen Maß davon ab, ob die jeweiligen Abbildungen der verschiedenen Bildserien mit zueinander entsprechenden Abbildungsparametern aufgenommen worden sind. Bei bekannten Systemen bzw. Verfahren sind beispielsweise Ausgabeeinheiten vorgesehen, mittels derer manuell einzustellende Abbildungsparameter des Objekts für einen Bediener ausgegeben werden, um möglichst identische bzw. reproduzierbare Eigenschaften der Abbildungen der Bildserien zu erzeugen.

Die bekannte Ausgabe von manuell einzustellenden Abbildungsparametern des Abbildungssystems verhindert jedoch nicht, dass Abbildungen oder ganze Bildserien mit falschen Abbildungsparametern aufgenommen bzw. erzeugt werden.

US 2011/090348 A1 offenbart ein Abbildungssystem aufweisend mehrere zu verwaltende Abbildungseinheiten insbesondere für Hochzeits- oder Studiofotographie. Das Abbildungssystem verwaltet Sätze von Einstellwerten, die jeweils Abbildungsparameter, insbesondere Verwaltungsparameter wie "Hochzeit" oder "Studio 1" oder einen zugeordneten Abbildungserfassungsparameter wie "Blende" oder "ISO" enthalten, und extrahiert bei der Abbildung verwendete Abbildungsparameter aus den jeweiligen Abbildungseinheiten. Das Abbildungssystem ist ausgebildet, die verwalteten Abbildungsparameter und die extrahierten Abbildungsparameter zu vergleichen und eine Warnung an einen Benutzer auszugeben, falls diese Parameter voneinander abweichen.

EP 1 913 870 A1 offenbart eine Vorrichtung zum Bestimmen von Indikationen, die bei der Diagnose von rheumatischen Erkrankungen helfen, umfassend mindestens einen Abschnitt zum Erfassen von Signalen von einem untersuchten Körper oder einem Teil davon, wobei der Erfassungsabschnitt eine Einheit zum Erfassen von Bildern durch Kernspinresonanz ist. In der Vorrichtung ist ferner ein Abschnitt zum Verarbeiten erfasster Bilder in Bezug auf Bilddaten und/oder Resonanzsignale integriert, wobei der Verarbeitungsabschnitt aus Bilddaten und/oder Resonanzsignalen Werte eines oder mehrerer verschiedener numerischer Parameter bestimmt..

WO 2006/116700A2 offenbart ein computerimplementiertes Qualitätssicherungssystem, das die Schritte des Abrufens der Qualitätssicherung und der unterstützenden Informationen aus einer Datenbank, das Empfangen von Informationen über technische Variablen aus einer Überwachung des Patienten und über Röntgengeräte bei der Durchführung einer Bildgebungsstudie sowie das Erzeugen eines Qualitätssicherungsscores nach der Bildgebungsstudie basierend auf den technischen Variablen und den Qualitätssicherungs- und unterstützenden Informationen umfasst, wobei der Score verwendet werden kann, um Trendanalysen durchzuführen und Klinikern, Radiologen und Abteilungen Empfehlungen für Schulungen und Feedback zu geben.

Ausgehend von diesem Stand der Technik ist es die Aufgabe der vorliegenden Erfindung, ein Verfahren sowie ein System vorzuschlagen, mit dem die Aufnahme bzw. Erzeugung von Abbildungen von Bildserien mit fehlerhaften manuell einzustellenden Abbildungsparametern vermieden wird.

Diese Aufgabe wird durch das Verfahren nach Anspruch 1 sowie das System nach Anspruch 10 gelöst, welche die Erfindung definieren.

Vorteilhafte Ausführungen des Verfahrens sowie des Systems sind Gegenstand der Unteransprüche.

Das Verfahren dient zur Erzeugung einer Serie von Abbildungen eines Objekts mit jeweils vorgegebenen Abbildungsparametern mittels eines teilautomatisierten Abbildungssystems, welches eine Datenbank, eine Abbildungseinheit, eine Recheneinheit, eine Speichereinheit und eine Ausgabeeinheit umfasst. Das Verfahren umfasst dabei die folgenden Verfahrensschritte:
In einem ersten Verfahrensschritt erfolgt die Auswahl einer Bildserienvorgabe umfassend eine Mehrzahl vordefinierter Sätze von Abbildungsparametern eines Objekts aus einer Datenbank. Eine entsprechende Bildserienvorgabe kann auch als Template bezeichnet werden. Beispielsweise und keinesfalls abschließend kann für den jeweiligen Satz einer Bildserienvorgabe ein bestimmter Teil des abzubildenden Körpers eine bestimmte räumliche Ausrichtung des abzubildenden Körpers bezüglich der Abbildungseinheit, eine bestimmte Haltung des abzubildenden Körpers sowie eine Vielzahl von Parametern einer Abbildungseinheit, wie beispielsweise Belichtungsdauer/Iso-Wert, Brennweite, Blitzeigenschaften und vieles mehr, als Abbildungsparameter hinterlegt bzw. gespeichert sein.

Wie bereits erläutert, wird das erfindungsgemäße Verfahren eingesetzt, um ausgehend von einer ersten Abbildungsserie einen Vergleich mit weiteren später aufgenommenen korrespondierenden Bildserien anzustellen. Dementsprechend kann das Verfahren zur Erzeugung einer Basis-Bildserie und zur späteren Erzeugung von Nachfolge-Bildserien verwendet werden. Im Fall einer Basis-Bildserie kann der erste Verfahrensschritt auch so ausgestaltet sein, dass die Auswahl der Bildserienvorgabe gespeichert wird. Dann kann beim Ausführen des Verfahrens zur Erzeugung einer Nachfolge-Bildserie lediglich eine mittelbare Auswahl der Bildserie auf Basis der gespeicherten Bildserienvorgabe vorgesehen sein. Mit anderen Worten ausgedrückt bedeutet dies, dass der erste Verfahrensschritt auch eine Eingabe umfassen kann, die festlegt, ob eine Basis-Bildserie oder eine Nachfolge-Bildserie erzeugt werden soll, wobei die Auswahl der Bildserienvorgabe entsprechend unmittelbar bei einer Basis-Bildserie und mittelbar bei einer Nachfolge-Bildserie erfolgt.

In einem weiteren Verfahrensschritt folgt die Ausgabe von zumindest einem manuell einzustellenden Abbildungsparameter des jeweiligen Satzes der Bildserienvorgabe auf der Ausgabeeinheit. Als Ausgabeeinheit kann beispielsweise ein Bildschirm dienen, der gegebenenfalls in Zusammenarbeit mit einer Recheneinheit dazu eingerichtet ist, Visualisierungen der manuell einzustellenden Abbildungsparameter zu erzeugen und auszugeben. Alternativ kann als Ausgabeeinheit auch eine akustische Ausgabeeinheit, wie beispielsweise ein Lautsprecher, vorgesehen sein. Mittels der so erzeugten Ausgaben wird der Benutzer auf die Abbildungsparameter hingewiesen, welche nicht im Rahmen der Teilautomatisierung des Abbildungssystems systemintern eingestellt werden können, sondern einer manuellen Einstellung bedürfen.

In einem folgenden Verfahrensschritt, der beispielsweise dadurch initiiert wird, dass der Benutzer die Einstellung der manuell einzustellenden Abbildungsparameter mittels einer entsprechenden Eingabe an einer Eingabeeinheit des Systems bestätigt, erfolgt die Erzeugung einer Abbildung mittels der Abbildungseinheit. Bei der Abbildungseinheit kann es sich beispielsweise um eine digitale Abbildungseinheit umfassend einen digitalen Bildsensor und eine Abbildungsoptik handeln. Dabei kann vorgesehen sein, dass das Abbildungssystem mit einer Vielzahl von kommerziell hergestellten Abbildungseinheiten, wie beispielsweise in Großserien hergestellten digitalen Spiegelreflexkameras, betrieben werden kann.

Im Anschluss an die Erzeugung der Abbildung wird diese mittels geeigneten Vorrichtungen des Systems an die Recheneinheit übertragen. Bei der Übertragung kann es sich beispielsweise um eine kabellose oder kabelgebundene Übertragung von digitalen Bilddaten bzw. digitalen Abbildungsdaten handeln.

Im folgenden Verfahrensschritt erfolgt die Auswertung der Abbildung hinsichtlich zumindest eines manuell eingestellten Abbildungsparameters mittels der Recheneinheit. Dabei kann die Recheneinheit die Abbildung bzw. die Bilddaten auf unterschiedliche Weise analysieren bzw. auswerten. Hierbei werden die tatsächlichen Bilddaten in der Form der einzelnen Bildpunkte und deren Anordnung zueinander ausgewertet. Zudem kann die Auswertung auch Daten berücksichtigen, die von der Abbildungseinheit erzeugt und mit den Abbildungsdaten übertragen werden, die direkt oder indirekt die bei der Abbildung vorherrschenden Abbildungsparameter der Abbildungseinheit betreffen. So erzeugen gängige Abbildungseinheiten beispielsweise für jede aufgenommene Abbildung auch Daten über Einstellungen der Abbildungseinheit, welche sich in der entsprechenden Abbildung selbst nicht widerspiegeln, wie beispielsweise die Brennweite einer Abbildungsoptik der Abbildungseinheit als Teil der Bilddaten.

Im Anschluss an die Auswertung wird in einem weiteren Verfahrensschritt eine Meldung mittels der Ausgabeeinheit ausgegeben, falls die vorangegangene Auswertung seitens der Recheneinheit ergibt bzw. ergeben hat, dass der zumindest eine manuell eingestellte Abbildungsparameter nicht mit der Ausgabe des einzustellenden Abbildungsparameters im entsprechenden vorangegangenen Verfahrensschritt übereinstimmt. So kann beispielsweise die Auswertung der Recheneinheit zu dem Ergebnis kommen, dass für den vorliegenden Satz der Bildserienvorgabe eine bestimmte Brennweite der Abbildungseinheit vorgegeben ist und diese als manuell einzustellender Abbildungsparameter ausgegeben wurde, wobei die analysierte bzw. ausgewertete Abbildung oder vielmehr die zugrundeliegenden Bilddaten jedoch direkt oder indirekt auf eine abweichende Brennweite der Abbildungseinheit schließen lassen. Die Abweichung kann mit der vorgesehenen Ausgabe einer Meldung durch die Ausgabeeinheit dem Benutzer zur Kenntnis gebracht werden.

Die vorausgehend beschriebenen Verfahrensschritte werden so, beginnend mit dem Verfahrensschritt der Ausgabe von zumindest einem manuell einzustellenden Abbildungsparameter, für jeden Satz der Bildserienvorgabe abgearbeitet.

Wie aus den beschriebenen einzelnen Verfahrensschritten bereits deutlich wird, ermöglicht das Verfahren insbesondere durch die Auswertung der Abbildung seitens der Recheneinheit und der anschließenden Ausgabe für den Fall, dass der zumindest eine manuell eingestellte Abbildungsparameter nicht dem ausgegebenen einzustellenden Abbildungsparameter entspricht, dass die Sätze von Abbildungen der entsprechenden Bildserienvorgaben bei jeder Abarbeitung, wie beispielsweise vor und nach einer Behandlung eines abzubildenden Körpers, mit identischen Abbildungsparametern, insbesondere hinsichtlich der manuell einzustellenden Abbildungsparameter, erzeugt werden, wodurch die Reproduzierbarkeit der Abbildungen und damit die Aussagekraft des Vergleichs der Abbildungen aus unterschiedlichen Bildserien enorm gesteigert wird.

In einer Weiterbildung des Verfahrens kann vorgesehen sein, dass in einem zusätzlichen Verfahrensschritt die Speicherung der Abbildung mittels Speichereinheit erfolgt. Dabei kann entweder vorgesehen sein, dass die Speicherung nur erfolgt, wenn die Auswertung der Recheneinheit keine Abweichung hinsichtlich der Vorgabe und der tatsächlichen manuell einzustellenden Abbildungsparameter ergeben hat. Alternativ kann jedoch auch vorgesehen sein, dass die Speicherung derart erfolgt, dass eine festgestellte Abweichung der manuell einzustellenden Abbildungsparameter von der Vorgabe mitabgespeichert wird. So kann sichergestellt werden, dass entweder nur Abbildungen mit korrekten manuell einzustellenden Abbildungsparametern gespeichert werden und dementsprechend für die Dokumentation zur Verfügung stehen oder aber dass die festgestellte Abweichung bei der Dokumentation und dem darauf basierenden Vergleich der Abbildungen mit dokumentiert werden und dadurch eine verfälschte Aussagekraft des Resultats durch den Vergleich der Abbildungen vermieden wird.

Zudem kann vorgesehen sein, dass das Verfahren einen zusätzlichen Verfahrensschritt umfasst, der sich an die Auswertung der Abbildung mittels der Recheneinheit anschließt und ein Wiederholen der vorangegangenen Verfahrensschritte, beginnend mit dem Verfahrensschritt der Ausgabe von zumindest einem manuell einzustellenden Abbildungsparameter für den jeweiligen Satz der Bildserienvorgabe vorsieht, für den Fall, dass die Auswertung ergibt, dass der zumindest eine manuell einzustellende Abbildungsparameter nicht mit der Ausgabe des entsprechenden Abbildungsparameters in dem vorangegangenen Verfahrensschritt übereinstimmt. Dies hat den Vorteil, dass so sichergestellt wird, dass für jeden Satz der Bildserienvorgabe eine Abbildung erstellt wird, in der die vorgegebenen manuell einstellbaren Abbildungsparameter auch tatsächlich vorliegen und somit eine Abbildung erzeugt und gespeichert werden kann, die zu einem aussagekräftigen Ergebnis beim Vergleich mit einer entsprechenden Abbildung einer früheren und/oder späteren Abbildung des gleichen Satzes der Bildserienvorgabe führt.

In einer weiteren Ausgestaltung des Verfahrens kann vorgesehen sein, dass das Speichern der Abbildung in einer Speichereinheit das automatische Erstellen eines Dateinamens und/oder von Metadaten der Abbildung umfasst. Gleichermaßen kann auch der Speicherort durch eine entsprechende Referenzierung der zu speichernden Datei der Abbildung automatisiert werden. Beispielsweise kann vorgesehen sein, dass der Dateiname und/oder die Metadaten auf Basis der Bildserienvorgabe, des entsprechenden Satzes der Bildserienvorgabe, einer Objektbezeichnung, eines Datums oder aufgrund verschiedener anderer dem System zugänglichen Informationen und/oder Abbildungs-parametern automatisch erzeugt wird.

Es ist zudem vorteilhaft, wenn das Verfahren einen weiteren Verfahrensschritt umfasst, der sich zwischen die Ausgabe von zumindest einem manuell einzustellenden Abbildungsparameter und die Erzeugung der entsprechenden Abbildung eingliedert und das Verwenden einer Einstellvorrichtung vorsieht, wobei mit der Einstellvorrichtung ein stufenlos einstellbarer Abbildungsparameter der Abbildungseinheit abgestuft manuell einstellbar ist. Beispielsweise ist die Brennweite von Abbildungsoptiken der Abbildungseinheiten bei einer ganz überwiegenden Vielzahl von kommerziell hergestellten Abbildungseinheiten bzw. Abbildungsoptiken manuell und stufenlos einstellbar. Mit der vorgeschlagenen Einstellvorrichtung, die dabei an der Abbildungseinheit und/oder der Abbildungsoptik der Abbildungseinheit temporär oder dauerhaft angeordnet ist, kann die Einstellung der Brennweite stufenweise vorgenommen werden. Eine derartige Verwendung einer Einstellvorrichtung hat den Vorteil, dass die vorgegebenen manuell einzustellenden Abbildungsparameter der Abbildungseinheit korrekt, insbesondere ohne geringfügige Abweichung, vom Benutzer eingestellt werden können.

In einer weiteren Ausgestaltung des Verfahrens ist vorteilhaft vorgesehen, dass in einem Verfahrensschritt, der der Erzeugung einer Abbildung vorgeschaltet ist, auch das Übertragen von zumindest einem ferngesteuert einstellbaren Abbildungsparameter von der Datenbank an eine Einheit des Abbildungssystems, insbesondere an die Abbildungseinheit und eine entsprechende Einstellung des Abbildungsparameters seitens der entsprechenden Einheit des Abbildungssystems vorgenommen wird. Durch die vorgeschlagene Übermittlung und Einstellung wird besonders vorteilhaft das Verfahren für teilautomatisierte Systeme angepasst. Mit anderen Worten ausgedrückt bedeutet dies, dass mittels des Verfahrens das Abbildungssystem wann immer möglich über entsprechende Schnittstellen ferngesteuert bzw. automatisch auf die entsprechenden Abbildungsparameter eingestellt wird und die manuelle Einstellung von Abbildungsparametern samt der initialen Ausgabe und der Auswertung der tatsächlichen manuellen Einstellung mittels der oben beschriebenen Rückkopplung bzw. Feedbackschleife nur noch für diejenigen Abbildungsparameter vorgenommen wird, welche zwangsläufig eine manuelle Einstellung seitens des Benutzers erfordern. So kann beispielsweise eine Belichtungszeit für die Abbildung über eine entsprechende Schnittstelle zwischen der Datenbank und der Abbildungseinheit an die Abbildungseinheit gesendet und von dieser entsprechend automatisch eingestellt werden.

In einer weiteren vorteilhaften Ausgestaltung umfasst der initiale Verfahrensschritt der Auswahl einer Bildserienvorgabe auch die Auswahl von Objektdaten aus einer weiteren Datenbank und/oder die Eingabe von Objektdaten an einer Eingabeeinheit des Abbildungssystems, wobei die Abbildungsparameter der Bildserienvorgabe in Abhängigkeit von den Objektdaten angepasst werden. Beispielsweise können in der weiteren Datenbank bzw. über die Eingabeeinheit Objektdaten, wie Größe, Geschlecht, Hauttyp, Gewicht, gespeichert sein bzw. eingegeben werden. Dabei kann vorgesehen sein, dass aufgrund einzelner oder mehrerer solcher Objektdaten anhand von geeigneten Algorithmen seitens der Recheneinheit die Abbildungsparameter für eine Bildserienvorgabe bzw. deren Sätze angepasst werden. Dadurch wird ermöglicht, dass alle Abbildungsparameter, insbesondere auch die manuell einzustellenden Abbildungsparameter, optimal an das abzubildende Objekt angepasst werden, wodurch auch der Aussagegehalt durch den Vergleich einander entsprechender Sätze von Abbildungen weiter verbessert werden kann.

Auch hier kann vorgesehen sein, dass die Anpassung der Abbildungsparameter bei der Durchführung des Verfahrens zur Erzeugung einer Basis-Bildserie erfolgt und bei der späteren Durchführung zur Erzeugung einer Nachfolge-Bildserie eine Übernahme der bereits angepassten und gegebenenfalls gespeicherten Abbildungsparameter stattfindet. Alternativ kann jedoch auch eine Anpassung bei jeder Durchführung des Verfahrens vorgesehen sein. Dies erlaubt beispielsweise eine Berücksichtigung von Veränderungen der Farbpigmente der Haut des abzubildenden Objekts.

In einer weiteren Ausgestaltung kann zudem vorgesehen sein, dass der initiale Verfahrensschritt der Auswahl einer Bildserienvorgabe über eine entsprechende Kombination von Ausgabe- und Eingabeschritten mittels der entsprechenden Eingabe- und Ausgabeeinheit ermöglicht, einzelne Sätze von Abbildungsparametern einer Bildserienvorgabe zu aktivieren bzw. zu deaktivieren, wobei die nachfolgenden Verfahrensschritte nur für die aktivierten Sätze von Abbildungsparametern der Bildserienvorgabe durchgeführt werden. Mit anderen Worten ausgedrückt bedeutet dies, dass es dem Nutzer durch eine entsprechende Eingabe und daraus resultierende Auswahl möglich gemacht wird, die Anzahl an Abbildungen und dementsprechend die Sätze von Abbildungsparametern einer Bildserienvorgabe auf die sinnvollen oder notwendigen Sätze zu beschränken. So kann beispielsweise ein Satz einer Bildserienvorgabe, der eine bestimmte räumliche Ausrichtung des Objekts bezüglich der Abbildungseinheit vorsieht, die der Benutzer insgesamt oder aufgrund der objektspezifischen Gegebenheiten für nicht sinnvoll oder nicht aussagekräftig erachtet, deaktiviert werden, um somit das gesamte Verfahren nicht unnötig aufzublähen bzw. auf aussagekräftige Abbildungen zu beschränken.

Eine derartige Veränderung der Bildserienvorgabe kann besonders vorteilhaft bei der Durchführung des Verfahrens zur Erzeugung einer Basis-Bildserie erfolgen und so gespeichert bzw. hinterlegt werden, dass die Veränderungen auch bei nachfolgenden Durchführungen des Verfahrens zur Erzeugung von Nachfolge-Bildserien erhalten bleiben.

Zudem vorteilhaft ist es, wenn im initialen Verfahrensschritt auch eine Erstellung einer neuen bzw. eigenen Bildserienvorgabe erfolgen kann. Dabei kann einerseits vorgesehen sein, dass die Bildserienvorgabe vollständig regeneriert wird. Andererseits kann jedoch auch vorgesehen sein, dass die Bildserienvorgabe durch Abwandlung einer bereits bestehenden Bildserienvorgabe erzeugt wird. Dabei kann ebenfalls über entsprechende Eingaben und Ausgaben an der Eingabeeinheit und der Ausgabeeinheit, beispielsweise unter Einbindung eines Editors einer graphischen Benutzeroberfläche, die Veränderung bzw. die Erzeugung neuer Bildserienvorgaben erfolgen.

Vorteilhaft ist die Erstellung nur global oder beim Durchführen des Verfahrens zur Erzeugung einer Basis-Bildserie bei entsprechender Übernahme der erzeugten Bildserienvorgabe für die nachfolgenden Durchführungen des Verfahrens vorgesehen.

Das erfindungsgemäße Abbildungssystem zum Erzeugen einer Serie von Abbildungen eines Objekts mit jeweils vorgegebenen Abbildungsparametern umfasst, wie bereits beschrieben, eine Datenbank, eine Abbildungseinheit, eine Recheneinheit, eine Speichereinheit und eine Ausgabeeinheit.

Dabei ist die Datenbank zur Speicherung von Bildserienvorgaben umfassend eine Mehrzahl vordefinierter Sätze von Abbildungsparametern eines Objekts eingerichtet. Die Eingabeeinheit ist zur Auswahl einer Bildserienvorgabe vorgesehen. Weiter ist die Abbildungseinheit zur Erzeugung von Abbildungen des Objekts und zur Übermittlung der Abbildungen an die Recheneinheit und/oder Speichereinheit und die Speichereinheit zur Speicherung der erzeugten Abbildungen eingerichtet. Zudem ist die Ausgabeeinheit zur Ausgabe von zumindest einem manuell einzustellenden Abbildungsparameter des jeweiligen Satzes einer ausgewählten Bildserienvorgabe eingerichtet, die Recheneinheit zudem zur Auswertung einer Abbildung hinsichtlich zumindest eines manuell einzustellenden Abbildungsparameters eingerichtet und die Ausgabeeinheit zur Ausgabe einer Meldung eingerichtet, falls die Auswertung mittels der Recheneinheit ergibt, dass der zumindest eine manuell einzustellende Abbildungsparameter nicht mit der Ausgabe des entsprechenden Abbildungsparameters auf der Ausgabeeinheit übereinstimmt.

Das vorgeschlagene Abbildungssystem ermöglicht so, dass für jede erzeugte Abbildung festgestellt wird, dass die manuell einzustellenden Abbildungsparameter korrekt eingestellt wurden, bevor die jeweilige Abbildung erzeugt wurde. Demnach kann mit dem vorgeschlagenen Abbildungssystem der Aussagegehalt beim Vergleich einander entsprechender Abbildungen verschiedener Bildserien dadurch erhöht werden, dass die einem jeweiligen Satz zugeordneten Abbildungen unterschiedlicher Bildserien identische manuell einzustellende Abbildungsparameter aufweisen bzw. mit identischen manuell einzustellenden Abbildungsparametern erzeugt wurden.

Weiter ist es besonders vorteilhaft, wenn die Recheneinheit dazu eingerichtet ist, Dateinamen und/oder Metadaten in Abhängigkeit einer Bildserienvorgabe, des Satzes der Bildserienvorgabe in Abhängigkeit von in einer weiteren Datenbank vorhandenen Objektdaten und/oder in Abhängigkeit von an einer Eingabeeinheit eingegebenen Information zu erzeugen und mit den Abbildungen in der Speichereinheit zu speichern.

Weiter ist vorteilhaft vorgesehen, dass das Abbildungssystem eine Einstelleinrichtung aufweist, die an der Abbildungseinheit angeordnet ist und eine abgestufte Einstellung eines mittels der Abbildungseinheit stufenlos einstellbaren Abbildungsparameters ermöglicht. Wie oben bereits beschrieben, ermöglicht eine derartige Einstellvorrichtung, dass die an der Abbildungseinheit stufenlos manuell einstellbaren Abbildungsparameter exakt, insbesondere auch ohne geringfügige Abweichungen, eingestellt werden.

Besonders vorteilhaft ist es dabei, wenn die Einstelleinrichtung derart an der Abbildungseinheit angeordnet ist, dass eine abgestufte Einstellung der Brennweite an einer Abbildungsoptik der Abbildungseinheit mit stufenlos verstellbarer Brennweite ermöglicht wird. Beispielsweise kann eine Einstellvorrichtung zwei konzentrisch zueinander angeordnete, zueinander drehbar gelagerte Ringelemente umfassen, die entsprechende Mittel zur Anordnung bzw. Befestigung an der Abbildungseinheit und/oder der Abbildungsoptik der Abbildungseinheit aufweisen.

Darüber hinaus können an den beiden Ringelementen zusammenwirkende Arretierelemente vorgesehen sein, die eine lösbare Arretierung der beiden Ringelemente in einer definierten Relativanordnung bezüglich einer durch die gemeinsame Mittelachse definierten Rotationsachse erlauben. Des Weiteren kann mit dem Befestigungsmittel eine definierte Anordnung hinsichtlich der Abbildungseinheit und/oder der Abbildungsoptik erreicht werden, um sicherzustellen, dass die stufenweise einstellbaren Positionen der Ringelemente zueinander mit einem manuell einzustellenden Abbildungsparameter, insbesondere mit einer bestimmten Brennweite, korrespondieren.

Zudem ist es besonders vorteilhaft, wenn das Abbildungssystem zumindest ein Übertragungsmittel umfasst, mit dem die Übertragung von zumindest einem ferngesteuert einstellbaren Abbildungsparameter an eine Einheit des Abbildungssystems, insbesondere an die Abbildungseinheit, erfolgt. Durch die vorgeschlagenen Übertragungsmittel, welche beispielsweise als Kombination aus Schnittstellen und/oder Kabelverbindungen gebildet werden können, kann sichergestellt werden, dass bei dem teilautomatisierten Abbildungssystem alle ferngesteuert einstellbaren Abbildungsparameter automatisch eingestellt werden und damit Fehler vermieden werden, welche die Vergleichbarkeit der resultierenden Abbildungen unterschiedlicher Bildserien des gleichen Objekts vermindern. Die Vermeidung von Fehlern hinsichtlich der Abbildungsparameter wird zudem durch die Auswertung der Abbildungen in Bezug auf die manuell einzustellenden Abbildungsparameter des Abbildungssystems verbessert.

In diesem Zusammenhang ist es weiter besonders vorteilhaft, wenn das Abbildungssystem zumindest ein Einstellmittel zum ferngesteuerten Einstellen eines Abbildungsparameters umfasst, wobei das Einstellmittel mit einem Übertragungsmittel verbunden ist, um den entsprechenden ferngesteuert einstellbaren Abbildungsparameter zu empfangen. Wie vorangehend bereits beschrieben, ermöglicht das Zusammenspiel der Übertragungsmittel und der jeweiligen Einstellmittel, dass einzelne Einheiten des Abbildungs-systems zumindest teilweise ferngesteuert auf die jeweils vorgesehenen Abbildungsparameter eingestellt werden.

Außerdem kann vorteilhaft vorgesehen sein, dass die Recheneinheit dazu eingerichtet ist, die Abbildungsparameter einer Bildserienvorgabe anhand von Objektdaten des abzubildenden Objekts anzupassen.

Nachfolgend werden einzelne Ausgestaltungen des Verfahrens sowie des Systems anhand der beispielhaften und lediglich schematisch dargestellten Zeichnungen erörtert. Es zeigen:
- Figur 1: eine Ablaufskizze des Verfahrens in einer ersten Ausgestaltung;
- Figur 2: ein Abbildungssystem gemäß einer ersten Ausgestaltung;
- Figur 3a: eine perspektivische Ansicht einer Einstellvorrichtung;
- Figur 3b: eine Frontalansicht einer Einstellvorrichtung gemäß Figur 3a;
- Figur 3c: einen Schnitt durch die Einstellvorrichtung der Figur 3b entlang der Ebene A-A;
- Figur 4a: eine perspektivische Ansicht einer Abbildungseinheit mit einer Einstellvorrichtung in einer ersten Einstellung;
- Figur 4b: eine perspektivische Ansicht einer Abbildungseinheit mit einer Einstellvorrichtung in einer zweiten Einstellung;
- Figur 4c: eine perspektivische Darstellung einer Abbildungseinheit mit Einstellvorrichtungen in einer dritten Einstellung;

- Figur 5a: eine erste beispielhafte Ausgabe einer Ausgabeeinheit umfassend eine Ausgabe manuell einzustellender Abbildungsparameter;
- Figur 5b: eine zweite beispielhafte Ausgabe einer Ausgabeeinheit umfassend eine Ausgabe manuell einzustellender Abbildungsparameter; und
- Figur 6: eine beispielhafte Ausgabe einer Ausgabeeinheit zur Veränderung einer in einer Datenbank gespeicherten Bildserienvorgabe.

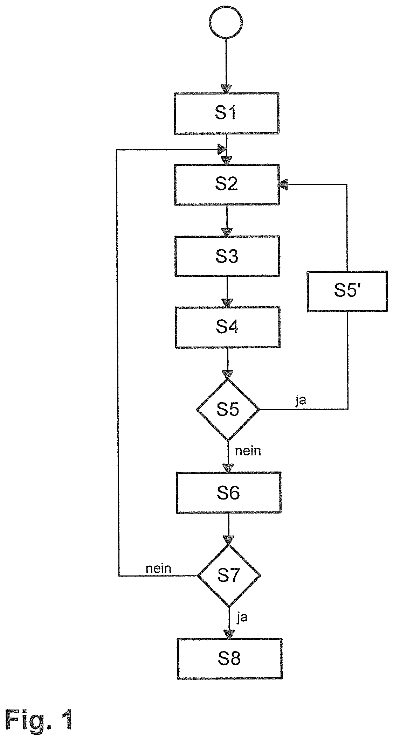

Figur 1 zeigt einen skizzierten Verfahrensablauf einer speziellen Ausgestaltung des erfindungsgemäßen Verfahrens. Nach dem Start wird ein erster Verfahrensschritt S1 ausgeführt, in dem zumindest die Auswahl einer entsprechenden Bildserienvorgabe aus einer Datenbank erfolgt. Zudem können abhängig davon, ob mit dem Verfahren eine Basis-Bildserie oder eine Nachfolge-Bildserie erzeugt wird, unmittelbar oder mittelbar Objektdaten aus einer weiteren Datenbank abgerufen oder direkt über eine Eingabeeinheit eingegeben werden. In diesem Fall kann zudem im Rahmen des Schritts S1 eine Anpassung der vorgegebenen Abbildungsparameter der Bildserienvorgabe anhand der Objektdaten erfolgen. Die Anpassung der Abbildungsparameter durch die Objektdaten erfolgt dabei entweder bei jeder Ausführung des Verfahrens aufgrund identischer Objektdaten oder aber lediglich einmalig beim ersten Ausführen des Verfahrens, wobei die angepassten Abbildungsparameter dann für weitere Durchläufe des Verfahrens gespeichert werden können. Dadurch wird sichergestellt, dass beispielsweise bei der Eingabe der Objektdaten an einer Eingabeeinheit die Vergleichbarkeit der Bildserien nicht durch eine fehlerhafte Eingabe bei einer oder mehreren erzeugten Bildserien vereitelt wird.

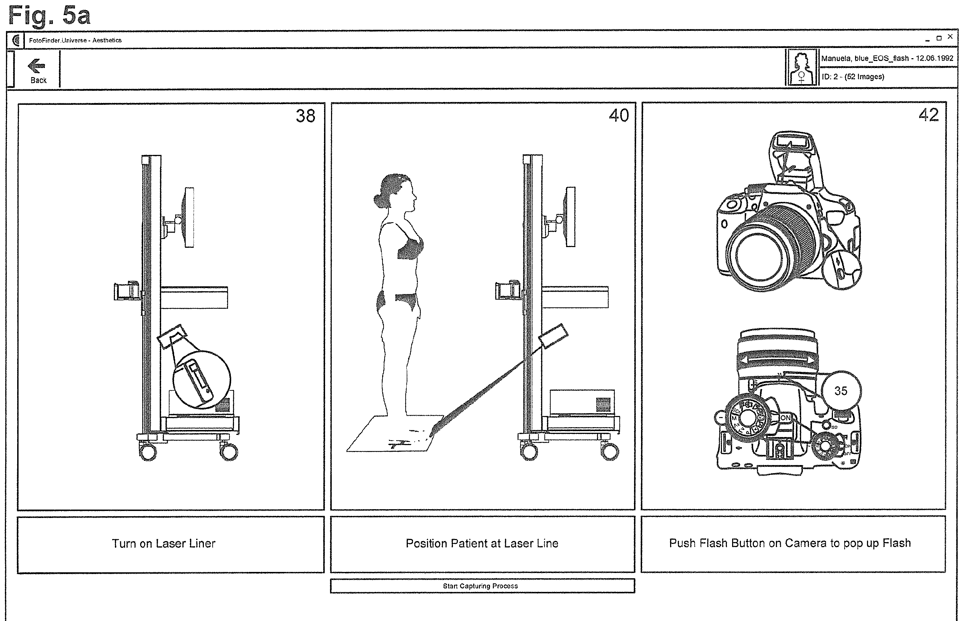

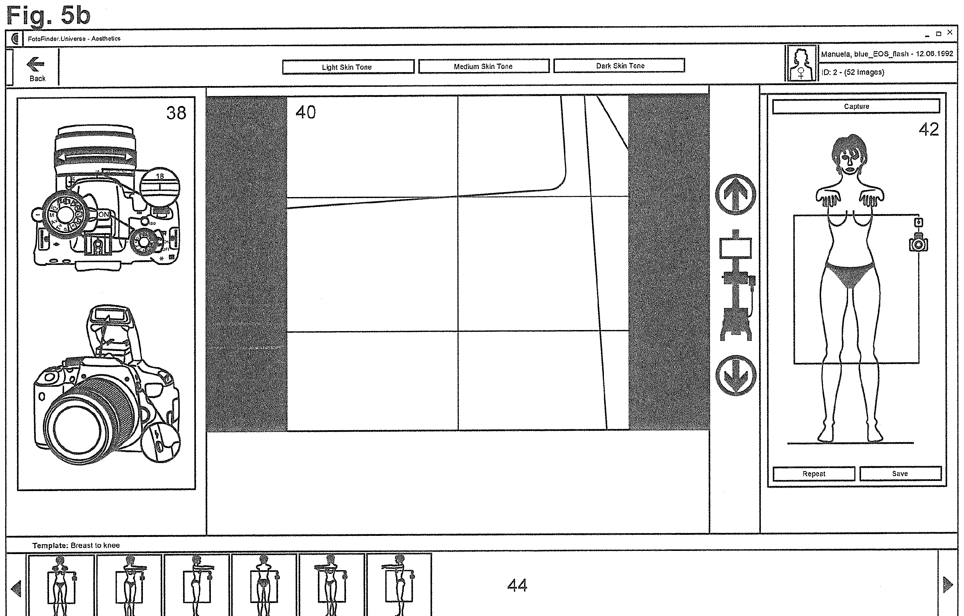

Im folgenden Verfahrensschritt S2 erfolgt zumindest die Ausgabe von zumindest einem manuell einzustellenden Abbildungsparameter des jeweiligen Satzes der Bildserienvorgabe auf einer Ausgabeeinheit, wie sie beispielhaft in den Figuren 5a und 5b dargestellt ist, die weiter unten noch ausführlicher erläutert werden. Zudem kann im Rahmen des Verfahrensschritts S2 auch vorgesehen sein, dass auch das Übertragen von zumindest einem ferngesteuert einstellbaren Abbildungsparameter von der Datenbank und/oder Recheneinheit an eine andere Einheit des Abbildungssystems, insbesondere an die Abbildungseinheit und die entsprechende Einstellung des Abbildungsparameters an der jeweiligen Einheit des Abbildungssystems, erfolgt.

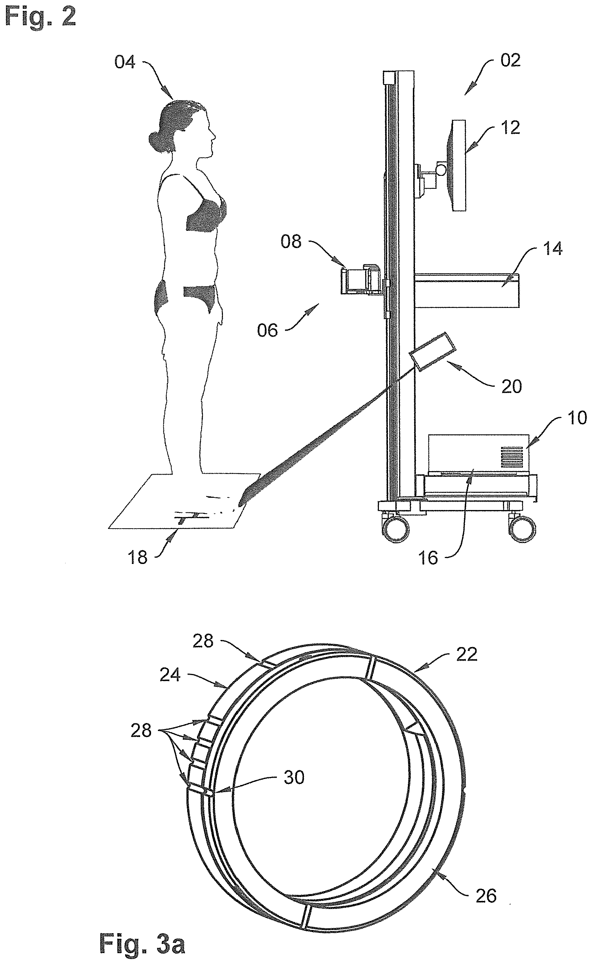

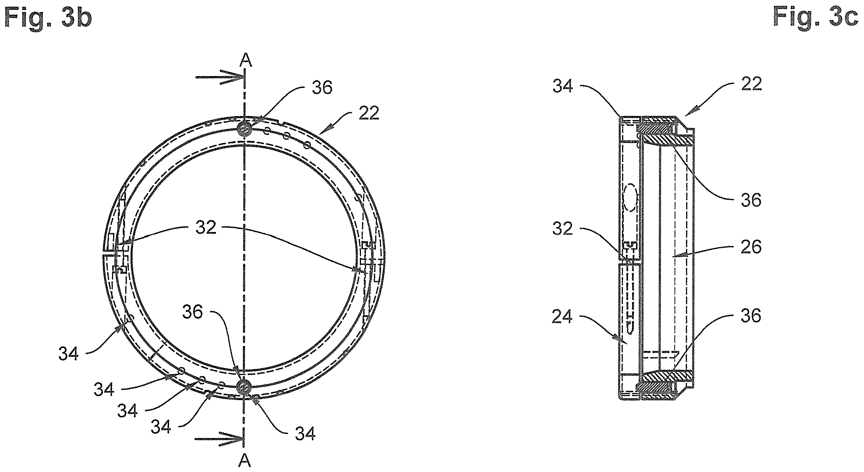

Zudem kann im Rahmen des Verfahrensschritts S2 eine Einstellvorrichtung verwendet werden, die zur abgestuften manuellen Einstellung des stufenlos manuell einstellbaren Abbildungsparameters der Abbildungseinheit dient. Eine genauere Beschreibung der Einstellvorrichtung sowie deren Verwendung finden sich in der nachfolgenden Beschreibung der Figuren 3, 4 und 5.

In dem folgenden Verfahrensschritt S3 erfolgt die Erzeugung einer Abbildung für den jeweiligen Satz der Bildserienvorgabe mittels der Abbildungseinheit. Im Anschluss daran wird die erzeugte Abbildung im Verfahrensschritt S4 an die Recheneinheit übertragen.

Anschließend erfolgt im Verfahrenschritt S5 die Auswertung der Abbildung hinsichtlich zumindest eines manuell eingestellten Abbildungsparameters mittels der Recheneinheit.

In der in Figur 1 dargestellten Ausgestaltung des Verfahrens ist vorgesehen, dass für den Fall, dass, wenn die Auswertung ergibt, dass der zumindest eine manuell eingestellte Abbildungsparameter nicht mit der Ausgabe des einzustellenden Abbildungsparameters im Verfahrensschritt S2 übereinstimmt, im Verfahrensschritt S5 einerseits eine entsprechende Ausgabe mittels der Ausgabeeinheit im Verfahrensschritt S5' erfolgt und zudem das Verfahren zu dem Verfahrensschritt S2 zurückspringt. Dementsprechend wird eine Schleife zwischen den Verfahrensschritten S2 und S5 für einen Satz einer Bildserienvorgabe solange durchlaufen, bis die Auswertung im Verfahrensschritt S5 keine Abweichungen zwischen dem zumindest einen ausgegebenen manuell einzustellenden Abbildungsparameter und dem tatsächlichen manuell eingestellten Abbildungs-parameter mehr feststellt.

Sobald keine derartige Abweichung als Resultat der Auswertung festgestellt wird, wird das Verfahren mit dem Verfahrensschritt S6 weitergeführt, indem die Speicherung der erzeugten Abbildung und eine entsprechende automatisierte Benennung anhand vorhandener Daten erfolgen kann.

In einem folgenden Verfahrensschritt S7 wird beispielsweise seitens der Recheneinheit geprüft, ob die zuletzt erzeugte und gespeicherte Abbildung einem letzten Satz der Bildserienvorgabe entspricht. Ist dies der Fall, so wird das Verfahren mit dem Verfahrensschritt S8 beendet. Wird andernfalls festgestellt, dass es sich bei der zuletzt erzeugten und gespeicherten Abbildung nicht um den letzten Satz der vorliegenden Bildserienvorgabe handelt, so wird das Verfahren im Verfahrensschritt S2 fortgeführt, wobei die oben beschriebenen Vorgänge des Verfahrensschritts S2 selbst, soweit nötig, für den nächsten bzw. den folgenden Satz der Bildserienvorgabe ausgeführt werden. Mit anderen Worten ausgedrückt bedeutet dies, dass jeweils neue manuell einzustellende Abbildungsparameter entsprechend dem neuen Satz der Bildserienvorgabe ausgegeben werden und gegebenenfalls eine entsprechende automatische Einstellung erfolgen kann.

Figur 2 zeigt ein erfindungsgemäßes Abbildungssystem 02 sowie ein abzubildendes Objekt 04. Das Abbildungssystem 02 dient zur zeitlich versetzten Erzeugung von mehreren Bildserien bzw. Serien von Abbildungen des Objekts oder eines Teils des Objekts 04. Das System 02 umfasst dabei eine Abbildungseinheit 06, welche beispielsweise als eine in Großserienfertigung hergestellte digitale Kamera ausgebildet ist. Die Abbildungseinheit 06 ist an einer Verstelleinheit 08 angeordnet, mit der die Abbildungseinheit 06 in der Höhe verstellt werden kann. Dabei sei beispielhaft darauf verwiesen, dass die Höhe der Abbildungseinheit auch ein Abbildungsparameter ist, der im Rahmen des erfindungsgemäßen Verfahrens manuell oder ferngesteuert eingestellt wird.

Das System umfasst ferner eine Recheneinheit 10, eine Ausgabeeinheit 12 und eine Eingabeeinheit 14. In die Recheneinheit 10 integriert ist zudem eine Speichereinheit 16, die zur Speicherung von zumindest einer Datenbank eingerichtet ist. Dabei ist vorgesehen, dass in der Speichereinheit 16 zumindest eine Datenbank vorliegt, die eine Mehrzahl von Bildserienvorgaben mit wiederum einer Mehrzahl vordefinierter Sätze von Abbildungsparametern eines Objekts umfasst. Darüber hinaus ist die Speichereinheit 16 zur Speicherung von erzeugten Abbildungen eingerichtet. Die Eingabeeinheit ist zur Auswahl einer Bildserienvorgabe eingerichtet. Die Abbildungseinheit 06 ist zur Erzeugung von Abbildungen des Objekts 04 und zur Übermittlung an die Recheneinheit 10 und/oder die Speichereinheit 16 eingerichtet.

Die Ausgabeeinheit 12 ist zur Ausgabe von zumindest einem manuell einzustellenden Abbildungsparameter des jeweiligen Satzes eingerichtet und die Recheneinheit 10 ist zudem zur Auswertung einer Abbildung hinsichtlich zumindest eines manuell einzustellenden Abbildungsparameters des Satzes der Bildserienvorgabe eingerichtet. Weiterhin ist die Ausgabeeinheit 12 zur Ausgabe einer Meldung eingerichtet, falls die Auswertung mittels der Recheneinheit 10 ergibt, dass der zumindest eine manuell einzustellende Abbildungsparameter nicht mit der Ausgabe des entsprechenden Abbildungsparameters auf der Ausgabeeinheit 12 übereinstimmt.

Weiter umfasst das System eine Fußmatte 18, auf der das Objekt 04 positioniert wird, wobei die Fußmatte 18 bzw. deren Positionierung gegenüber dem Abbildungssystem 02 mittels einer Messeinrichtung 20 erfasst werden kann. Die Messeinrichtung 20 kann als optische Einrichtung, insbesondere als Lasermesseinrichtung, ausgeführt sein. Es kann vorgesehen sein, dass bei jedem Ausführen des erfindungsgemäßen Verfahrens in einem initialen Verfahrensschritt die mit der Messeinrichtung 20 festgestellte Anordnung der Fußmatte 18 gegenüber dem Abbildungssystem dazu verwendet wird, die entsprechenden Abbildungsparameter aller Sätze von Bildserienvorgaben anzupassen, bis eine erneute veränderte Ausrichtung zwischen der Messeinrichtung 20 und der Fußmatte 18 festgestellt wird.

Figur 3a zeigt eine perspektivische Darstellung einer Einstellvorrichtung 22. Die Einstellvorrichtung 22 umfasst ein erstes Ringelement 24 sowie ein zweites Ringelement 26, wobei die beiden Ringelemente 24 und 26 koaxial und zueinander drehbar miteinander in Verbindung stehen. Das erste Ringelement 24 weist zudem eine Mehrzahl von Auswahlmarkierungen 28 auf. Auf dem zweiten Ringelement 26 ist zudem eine Einstellmarkierung 30 angeordnet. Die Einstellvorrichtung 22 ist so ausgebildet, dass sie an einer Abbildungseinheit 06 bzw. einer Abbildungsoptik einer Abbildungs-einheit 06 derart angeordnet werden kann, dass eine abgestufte Einstellung der Brennweite an einer Abbildungseinheit 06 mit stufenlos verstellbarer Brennweite ermöglicht wird.

Diese Funktionsweise der Einstellvorrichtung 22 ist aus der Figur 3b ersichtlich. Diese zeigt, dass die Einstellvorrichtung 22 Befestigungsmittel 32 im ersten und/oder zweiten Ringelement 24 bzw. 26 aufweist. Die Befestigungsmittel 32 gemäß der Figur 3b sind dabei als Schrauben und entsprechende Gewindelöcher ausgestaltet, die eine Beeinflussung des Innendurchmessers der Ringelemente und einen dadurch resultierenden Klemmsitz auf Teilen der Abbildungseinheit bzw. deren Abbildungsoptik ermöglichen. Alternativ zu den Befestigungsmitteln 32 kann die Einstellvorrichtung 22 jedoch auch derart ausgestaltet sein, dass eine Selbstsicherung der Einstellvorrichtung 22 bzw. der beiden Ringelemente 24 und 26 an der Abbildungseinheit erfolgt. Besonders bevorzugt ist es weiter, wenn die Einstellvorrichtung Kalibrierungsmittel aufweist, die ermöglichen, dass die Relativanordnung der Ringelemente, in der die Einstellmarkierung 30 jeweils in Verlängerung zu einer Auswahlmarkierung 28 angeordnet ist, einem bestimmten Wert der Brennweite der Abbildungseinheit entspricht. Damit wird sichergestellt, dass eine Relativbewegung der Ringelemente 24 und 26 zu einer bestimmten Brennweite der Abbildungseinheit führt, wobei die Einstellmarkierung 30 und eine Auswahlmarkierung 28 in Flucht zueinander angeordnet sind.

Um weiter zu ermöglichen, dass mittels der Einstellvorrichtung 22 der manuell einzustellende Abbildungsparameter der Brennweite der Abbildungseinheit 06 stufenweise eingestellt werden kann, obwohl die Abbildungseinheit 06 mit einer stufenlos verstellbaren Abbildungsoptik 07 ausgestattet ist, können die Ringelemente 24 und 26 mit zusammenwirkenden Arretierelementen 34 und 36 versehen sein. Die Figur 3b zeigt beispielsweise, dass eines der Ringelemente 24 und 26 eine Mehrzahl von Vertiefungen 34 aufweist und das jeweils andere Ringelement 24 oder 26 entsprechende Eingriffselemente 36 aufweist, die so angeordnet sind, dass sie bei einer Relativbewegung der Ringelemente 24 und 26 zueinander in die Ausnehmungen 34 lösbar eingreifen können.

Beispielsweise können die Eingriffselemente 36 als federbelastete Bolzen ausgeführt sein, wobei die Bolzen mittels der Federspannung oder einer vergleichbaren Krafteinwirkung durch ein elastisches Element gegen das die Ausnehmungen 34 umfassende Ringelement 24 oder 26 gedrückt werden, so dass, wenn, als Folge einer Relativbewegung der Ringelemente 24 und 26 zueinander, die Eingriffselemente bzw. die Bolzen 36 und die Ausnehmungen 34 in Überdeckung zueinander gebracht werden, ein Einrasten bzw. ein Arretieren der Ringelemente 24, 26 erfolgt. Dabei kann vorgesehen sein, dass die Eingriffselemente 36 und die Ausnehmungen 34 so zueinander angeordnet sind, dass ein Arretieren oder Einrasten der Ringelemente 24 und 26 in den Relativpositionen erfolgt, in denen jeweils eine flüchtige Anordnung der Auswahlmarkierungen 28 zur Einstellungsmarkierung 30 vorliegt.

In Figur 3c ist der Schnitt durch die Ebene A-A der Einstellvorrichtung 22 der Figur 3b gezeigt. In der Figur 3b wird abermals das Befestigungselement 32 dargestellt, welches eine Befestigung der Einstellvorrichtung 22 an der Abbildungseinheit 06 erlaubt. Zudem sind diametral gegenüberliegende Ausnehmungen 34 im ersten Ringelement 24 und mit diesen zusammenwirkende Eingriffselemente 36 im zweiten Ringelement 26 dargestellt.

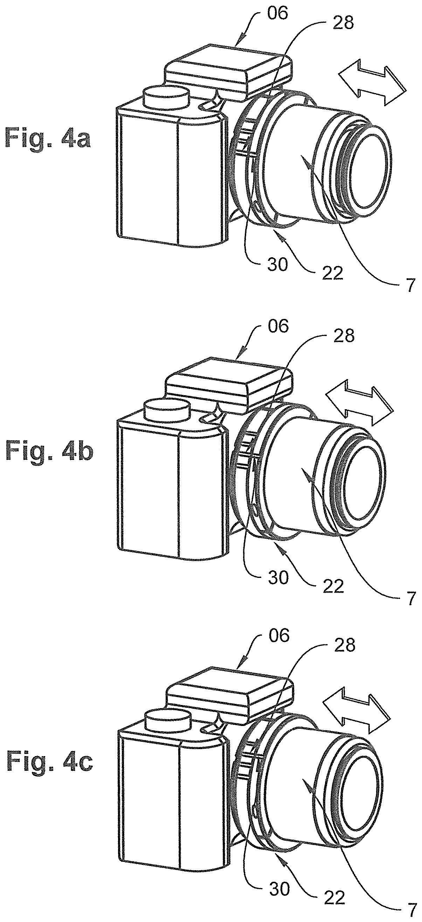

Die Figuren 4a bis 4c zeigen eine Abbildungseinheit, wie sie bei dem erfindungsgemäßen Verfahren bzw. dem erfindungsgemäßen Abbildungssystem zum Einsatz kommen kann. Die Abbildungseinheit 06 umfasst dabei eine Abbildungsoptik 07. Zudem umfasst die Abbildungseinheit 06 eine Einstellvorrichtung 22, die es ermöglicht, eine stufenweise Einstellung der Brennweite als Abbildungsparameter vorzunehmen, obwohl es sich bei der Abbildungsoptik 07 um eine Optik mit einer stufenlos einstellbaren Brennweite handelt. Wie in den Figuren 4a bis 4c gezeigt, wird zur Einstellung einer bestimmten Brennweite der Abbildungsoptik 07 der Abbildungseinheit 06 die Einstellmarkierung 30 in Flucht zu einer der Auswahlmarkierungen 28 positioniert, wodurch gleichzeitig, wie mit Bezug auf Figur 3 beschrieben, ein Einrasten bzw. eine Arretierung der Einstellvorrichtung 22 erreicht wird.

Dabei kann vorgesehen sein, dass die Auswahlmarkierungen 28 unterschiedliche Ausgestaltungen, wie beispielsweise unterschiedliche farbliche Gestaltung, aufweisen, um eine leichte Identifizierung der jeweils richtigen Einstellposition der Einstellvorrichtung 22 zu erlauben. Beispielsweise kann dementsprechend im erfindungsgemäßen Verfahren vorgesehen sein, dass die Ausgabe des zumindest einen manuell einzustellenden Abbildungsparameters die farbliche Ausgestaltung der Auswahlmarkierung 28 umfasst, so dass der Benutzer einfach ableiten kann, wie die Einstellmarkierung der Einstellvorrichtung 30 bezüglich der Auswahlmarkierungen 28 zu positionieren ist.

Figur 5a zeigt eine beispielhafte Ausgabe mittels der Ausgabeeinheit 16, welche die Ausgabe im Verfahrensschritt S2 des erfindungsgemäßen Verfahrens näher beschreibt.

Die Darstellung der Figur 5a zeigt im Wesentlichen ein linkes Segment 38, ein mittleres Segment 40 und ein rechtes Segment 42. Die Elemente 38 bis 42 sind dabei insgesamt Teil einer Ausgabe, welche dem Bediener die Bedienung des Abbildungssystems 02 bzw. die Durchführung des Verfahrens erleichtern soll. Im linken Element 38 sowie im mittleren Element 40 sind jeweils bildliche als auch schriftliche Anweisungen an den Bediener dargestellt, um die Durchführung des Verfahrens zu initiieren.

Im rechten Element 42 erfolgt zudem eine Ausgabe von zwei manuell einzustellenden Abbildungsparametern. Der erste manuell einzustellende Abbildungsparameter ist dabei die Aktivierung des Blitzes der Abbildungseinheit. Der zweite manuell einzustellende Abbildungsparameter ist die Einstellung der Brennweite auf 35 mm. Der erste manuell einzustellende Abbildungsparameter ist im rechten Element 42 sowohl grafisch als auch schriftlich wiedergegeben. Der zweite manuell einzustellende Abbildungsparameter ist im rechten Element 42 der Figur 5a lediglich bildlich dargestellt. Durch die Ausgabe, wie sie in der Figur 5a beispielhaft dargestellt ist, wird die korrekte Bedienung des Abbildungssystems erleichtert, weil zusammen mit dem erfindungsgemäßen Verfahren sichergestellt wird, dass vergleichbare bzw. reproduzierbare Bildserien mittels des Abbildungssystems 02 generiert werden.

Die Figur 5b zeigt eine alternative Ausgabe, wie sie ebenfalls während des Verfahrensschritts S2 auf einer Ausgabeeinheit 16 des Abbildungssystems 02 dargestellt werden kann. Neben einem linken Element 38, einem mittleren Element 40 und einem rechten Element 42 umfasst die gezeigte Darstellung auch ein unteres Element 44. Das linke Element 38 der Figur 5b entspricht im Wesentlichen dem rechten Element 42 der Figur 5a und dient zur Ausgabe von zwei manuell einzustellenden Abbildungsparametern, nämlich der Aktivierung des Blitzes der Abbildungseinheit und der Einstellung der Brennweite der Abbildungseinheit. Das rechte Element 42 umfasst eine weitere Ausgabe eines manuell einzustellenden Abbildungsparameters. Dabei handelt es sich um den zu wählenden Bereich des abzubildenden Objekts 04.

Im rechten Teil des rechten Elements 42 ist dazu eine bildliche Abbildung eines beispielhaften abzubildenden Objekts 04 und eine entsprechende Darstellung des zu wählenden Teilbereichs dargestellt. Im linken Teil des rechten Elements 42 ist zudem eine schematische Darstellung des Abbildungssystems 02 dargestellt.

Die oberhalb und unterhalb dieser schematischen Darstellung angeordneten Pfeile können unterschiedliche Funktionen haben. Einerseits können sie lediglich als Hinweis an den Benutzer dienen, dass die Abbildungseinheit 06 mit der Verstelleinrichtung 08 so verfahren werden muss, dass der im rechten Teil des rechten Elements 42 dargestellte Teil des abzubildenden Objekts von der Abbildungseinheit 06 erfasst wird, was beispielsweise durch das Kontrollfenster im mittleren Element 40 überprüft werden kann, in dem der tatsächliche Abbildungsbereich der Abbildungseinheit 06 wiedergegeben wird.

Alternativ kann auch vorgesehen sein, dass die Pfeile im rechten Element 42 der Figur 5b als Bedienelemente ausgeführt sind, mittels derer bei einer entsprechenden Benutzereingabe die Abbildungseinheit 06 des Abbildungssystems 02 über die Verstelleinrichtung 08 des Abbildungssystems 02 verstellt werden kann. Auf diese Möglichkeit wird deshalb explizit hingewiesen, weil dadurch klargestellt wird, was im Sinne der vorliegenden Offenbarung als manuell einzustellender Abbildungsparameter gilt. Ein manuell einzustellender Abbildungsparameter kann auch, wie im letztbeschriebenen Beispiel der Figur 5b, mittelbar von einem Benutzer unter Zuhilfenahme entsprechender technischer Einstellmittel eingestellt werden. Ausschlaggebend als Einordnung als manuell einzustellender Abbildungsparameter ist dabei, dass zumindest irgendeine Art der aktiven Einstellung seitens eines Benutzers erforderlich ist.

Das untere Element 44 in der beispielhaften Ausgabe der Figur 5b zeigt eine Bildserienvorgabe, umfassend eine Mehrzahl vordefinierter Sätze von Abbildungsparametern des Objekts. Die unterschiedlichen Sätze von Abbildungsparametern werden im Beispiel des unteren Elements 44 der Figur 5b hauptsächlich durch den Abbildungsparameter der unterschiedlichen räumlichen Ausrichtung des Objekts bezüglich der Abbildungseinheit verdeutlicht. Darüber hinaus kann jedoch auch vorgesehen sein, dass für den jeweiligen Satz darüber hinaus noch eine Vielzahl von nicht aus den Piktogrammen im unteren Element 44 hervorgehenden Abbildungsparametern zueinander unterschiedlich sind.

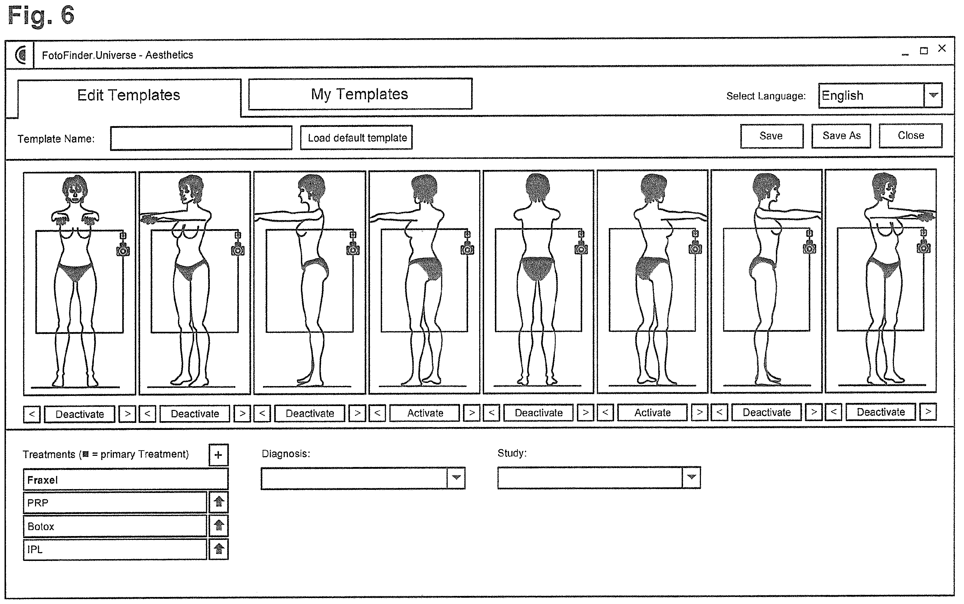

Figur 6 zeigt eine beispielhafte Ausgabe einer Ausgabeeinheit 16 des Abbildungssystems 02, welches eine Beeinflussung bzw. Abänderung einer Bildserienvorgabe beschreibt. Vor dem erstmaligen Durchführen des Verfahrens für ein bestimmtes Objekt bzw. beim Durchführen des Verfahrens zur Erzeugung einer Basis-Bildserie kann eine entsprechende Bildserienvorgabe angepasst werden. Beispielsweise können einzelne Sätze von Abbildungsparametern aktiviert oder deaktiviert werden, um das Verfahren zu verschlanken, wie es aus der beispielhaften Darstellung der Figur 6 hervorgeht. Dementsprechend werden bei der nachfolgenden Durchführung des Verfahrens zur Erzeugung einer Nachfolge-Bildserie nur Abbildungen für diejenigen Sätze von Abbildungsparametern erzeugt und ausgewertet, die in der Bildserienvorgabe aktiviert sind.

Mittels einer vergleichbaren Ausgabe, wie in Figur 6 dargestellt, und einer entsprechenden Interaktion bzw. einem Eingabe- und Ausgabedialog mit dem Benutzer kann dieser selbstständig neue Bildserienvorgaben erzeugen.

## Patentansprüche

1. Verfahren zum Erzeugen einer Serie von Abbildungen eines als menschlicher oder tierischer Körper definierten Objekts mit jeweils vorgegebenen Abbildungs-parametern mittels eines teilautomatisierten Abbildungssystems (02) zur Dokumentation eines Ausgangszustands des Körpers vor einem medizinischen oder ästhetischen Eingriff oder einer Behandlung sowie der weiteren Entwicklung des Körpers nach dem Eingriff oder der Behandlung, umfassend eine Datenbank, eine Abbildungseinheit (06), eine Recheneinheit (10), eine Speichereinheit (16) und eine Ausgabeeinheit (12) mit den Verfahrensschritten:
a) Auswahl einer Bildserienvorgabe umfassend eine Mehrzahl vordefinierter Sätze von Abbildungsparametern eines Objekts aus der Datenbank (S1), wobei der jeweilige Abbildungsparametersatz einen bestimmten Teil des abzubildenden Körpers, eine bestimmte räumliche Ausrichtung des abzubildenden Körpers bezüglich der Abbildungseinheit, eine bestimmte Haltung des abzubildenden Körpers und/oder eine Vielzahl von Parametern einer Abbildungseinheit als Abbildungsparameter aufweist;
- Erfassung der Anordnung einer Fußmatte (18) mittels einer Messeinrichtung (20) des Abbildungssystems (02) und Verwendung der festgestellten Anordnung der Fußmatte (18) gegenüber dem Abbildungssystem (02) zur Anpassung der Sätze von Abbildungsparametern der Bildserienvorgabe;
b) Ausgabe von zumindest einem manuell einzustellenden Abbildungsparameter des jeweiligen Satzes auf der Ausgabeeinheit (12) (S2) und Positionieren des Objekts auf der Fußmatte (18) entsprechend des wenigstens einen ausgegebenen Abbildungsparameters;
c) Erzeugen einer Abbildung mittels der Abbildungseinheit (06) (S3);
d) Übertragen der Abbildung an die Recheneinheit (10) (S4);
e) Auswertung der Abbildung hinsichtlich zumindest eines manuell eingestellten Abbildungsparameters mittels der Recheneinheit (10) (S5), wobei für die Auswertung Bilddaten der Abbildung in Form einzelner Bildpunkte und deren Anordnung zueinander ausgewertet werden;
f) Ausgabe einer Meldung mittels der Ausgabeeinheit (12) (S5'), falls die Auswertung ergibt, dass der zumindest eine manuell eingestellte Abbildungsparameter nicht mit der Ausgabe des einzustellenden Abbildungsparameters im Verfahrensschritt b) übereinstimmt; und
g) Wiederholen der Verfahrensschritte b) bis f) für jeden Satz der Bildserienvorgabe.

2. Verfahren nach Anspruch 1,
**dadurch gekennzeichnet,**
**dass** der Verfahrensschritt g) auch das Speichern der Abbildung in der Speichereinheit (16) umfasst.

3. Verfahren nach Anspruch 1 oder 2,
**gekennzeichnet durch**
einen Verfahrensschritt f1) umfassend das Wiederholen der Verfahrensschritte b) bis e) für den jeweiligen Satz der Bildserienvorgabe, falls die Auswertung ergibt, dass der zumindest eine manuell eingestellte Abbildungsparameter nicht mit der Ausgabe des einzustellenden Abbildungsparameters im Verfahrensschritt b) übereinstimmt.

4. Verfahren nach einem der Ansprüche 1 bis 3,
**dadurch gekennzeichnet,**
**dass** das Speichern der Abbildung in einer Speichereinheit (16) das automatische Erstellen eines Dateinamens und/oder von Metadaten der Abbildung umfasst.

5. Verfahren nach einem der Ansprüche 1 bis 4,
**gekennzeichnet durch**
einen Verfahrensschritt b1) umfassend das Verwenden einer Einstellvorrichtung (22) zur abgestuften manuellen Einstellung eines stufenlos einstellbaren Abbildungsparameters der Abbildungseinheit (06).

6. Verfahren nach einem der Ansprüche 1 bis 5,
**dadurch gekennzeichnet,**
**dass** der Verfahrensschritt b) auch das Übertragen von zumindest einem ferngesteuert einstellbaren Abbildungsparameter von der Datenbank an eine Einheit des Abbildungssystems (02), insbesondere die Abbildungseinheit (06), und die entsprechende Einstellung des Abbildungsparameters seitens der Einheit, insbesondere der Abbildungseinheit (06), umfasst.

7. Verfahren nach einem der Ansprüche 1 bis 6,
**dadurch gekennzeichnet,**
**dass** der Verfahrensschritt a) die Auswahl von Objektdaten aus einer weiteren Datenbank und/oder die Eingabe von Objektdaten an einer Eingabeeinheit (14) des Abbildungssystems (02) umfasst, wobei die Abbildungsparameter der Bildserienvorgabe in Abhängigkeit von den Objektdaten angepasst werden.

8. Verfahren nach eine der vorhergehenden Ansprüche, wobei für die Auswertung im Verfahrensschritt e) von der Abbildungseinheit auch Daten berücksichtigt werden, die von der Abbildungseinheit erzeugt und mit den Abbildungsdaten übertragen werden, die direkt oder indirekt die bei der Abbildung vorherrschenden Abbildungsparameter betreffen.

9. Verfahren nach einem der vorhergehenden Ansprüche, wobei das Verfahren die Erzeugung einer Abbildungsserie durch Ausführung der Schritte a) bis g) als Basis-Bildserie vor einem medizinischen oder ästhetischen Eingriff oder einer Behandlung sowie die Erzeugung einer Nachfolge-Bildserie durch Ausführung der Schritte a) bis g) nach dem medizinischen oder ästhetischen Eingriff oder einer Behandlung umfasst, wobei der erste Verfahrensschritt a) bei Erzeugung der Basis-Bildserie derart ausgestaltet ist, dass die Auswahl der Bildserienvorgabe gespeichert wird und beim Erzeugen der Nachfolge-Bildserie eine mittelbare Auswahl der Bildserie auf Basis der gespeicherten Bildserienvorgabe erfolgt.

10. Abbildungssystem (02) zum Erzeugen einer Serie von Abbildungen eines als menschlicher oder tierischer Körper definierten Objekts mit jeweils vorgegebenen Abbildungsparametern zur Dokumentation eines Ausgangszustands des Körpers vor einem medizinischen oder ästhetischen Eingriff oder einer Behandlung sowie der weiteren Entwicklung des Körpers nach dem Eingriff oder der Behandlung, umfassend eine Datenbank, eine Abbildungseinheit (06), eine Recheneinheit (10), eine Speichereinheit (16) und eine Ausgabeeinheit (12), wobei die Datenbank zumindest eine Bildserienvorgabe umfassend eine Mehrzahl vordefinierter Sätze von Abbildungsparametern eines Objekts umfasst und die Eingabeeinheit (14) zur Auswahl einer Bildserienvorgabe eingerichtet ist, wobei der jeweilige Abbildungsparametersatz einen bestimmten Teil des abzubildenden Körpers, eine bestimmte räumliche Ausrichtung des abzubildenden Körpers bezüglich der Abbildungseinheit, eine bestimmte Haltung des abzubildenden Körpers und/oder eine Vielzahl von Parametern einer Abbildungseinheit als Abbildungsparameter aufweist, die Abbildungs-einheit (06) zur Erzeugung von Abbildungen des Objekts und zur Übermittlung an die Recheneinheit (10) und/oder Speichereinheit (16) eingerichtet ist und die Speichereinheit (16) zur Speicherung von erzeugten Abbildungen eingerichtet ist, wobei die Ausgabeeinheit (12) zur Ausgabe von zumindest einem manuell einzustellenden Abbildungsparameter des jeweiligen Satzes eingerichtet ist, und das Abbildungs-system (02) eine Fußmatte (18) zur Positionierung des Objekts entsprechend des wenigstens einen ausgegeben Abbildungsparameters aufweist; wobei die Recheneinheit (10) zur Auswertung einer Abbildung hinsichtlich zumindest eines manuell einzustellenden Abbildungsparameters des Satzes durch Auswertung von Bilddaten der Abbildung in Form einzelner Bildpunkte und deren Anordnung zueinander eingerichtet ist, und
die Ausgabeeinheit (12) zur Ausgabe einer Meldung eingerichtet ist, falls die Auswertung mittels der Recheneinheit (10) ergibt, dass der zumindest eine manuell eingestellte Abbildungsparameter nicht mit der Ausgabe des manuell einzustellenden Abbildungsparameters auf der Ausgabeeinheit (12) übereinstimmt, wobei
das Abbildungssystem (02) ferner eine Messeinrichtung (20) umfasst, welche zur Erfassung der Positionierung der Fußmatte (18) gegenüber dem Abbildungssystem ausgebildet ist, und wobei das Abbildungssystem (02) derart ausgebildet ist, die festgelegte Anordnung der Fußmatte (18) gegenüber dem Abbildungssystem (02) dazu zu verwenden, die Sätze von Abbildungsparametern der Bildserienvorgabe anzupassen.

11. Abbildungssystem (02) nach Anspruch 10,
wobei die Recheneinheit (10) dazu eingerichtet ist Dateinamen und/oder Metadaten in Abhängigkeit der Bildserie und/oder des Satzes der Bildserie und/oder Objektdaten und/oder an einer Eingabeeinheit (14) eingegebener Informationen zu erzeugen und mit den Abbildungen in der Speichereinheit (16) zu speichern.

12. Abbildungssystem (02) nach einem der Ansprüche 10 oder 11,
wobei das Abbildungssystem (02) eine Einstellvorrichtung (22) aufweist, die an der Abbildungseinheit (06) angeordnet ist und eine abgestufte Einstellung eines stufenlos einstellbaren Abbildungsparameters ermöglicht.

13. Abbildungssystem (02) nach Anspruch 12,
wobei die Einstellvorrichtung (22) so an der Abbildungseinheit (06) angeordnet ist, dass eine abgestufte Einstellung der Brennweite an einer Abbildungsoptik der Abbildungseinheit (06) mit stufenlos einstellbarer Brennweite ermöglicht wird.

14. Abbildungssystem (02) nach einem der Ansprüche 10 bis 13,
wobei das Abbildungssystem (02) zumindest ein Übertragungsmittel umfasst, mit dem die Übertragung von zumindest einem ferngesteuert einstellbaren Abbildungs-parameter an eine Einheit des Abbildungssystems (02) erfolgt.

15. Abbildungssystem (02) nach Anspruch 14,
wobei das Abbildungssystem (02) zumindest ein Einstellmittel zur ferngesteuerten Einstellung eines Abbildungsparameters umfasst, wobei das Einstellmittel mit einem Übertragungsmittel verbunden ist, um den entsprechenden ferngesteuert einstellbaren Abbildungsparameter zu empfangen.

16. Abbildungssystem (02) nach einem der Ansprüche 10 bis 15,
wobei die Recheneinheit (10) dazu eingerichtet ist, die Abbildungsparameter einer Bildserienvorgabe anhand von Objektdaten anzupassen.

## Claims

1. A method for generating a series of images of objects defined as a human or animal body, each object having predetermined image parameters, by means of a partly automated imaging system (02) for documenting an initial state of the body before a medical or aesthetic procedure or a treatment and for documenting the further development of the body after the procedure or treatment, the imaging system (02) comprising a data bank, an imaging unit (06), a computing unit (10), a storage unit (16) and an output unit (12), the method comprising the following steps:
a) selecting a picture series presetting comprising a plurality of predefined groups of image parameters of an object from a databank (S1), the corresponding imaging parameter group having a certain part of the body to be imaged, a certain spatial orientation of the body to be imaged with respect to the imaging unit, a certain posture of the body to be imaged and/or a plurality of parameters of an imaging unit as an imaging parameter;
― detecting the arrangement on a floor mat (18) by means of a measuring device (20) of the imaging system (02) and using the determined arrangement of the floor mat (18) with respect to the imaging system (02) for adjusting the groups of imaging parameters of the image series presetting;
b) outputting at least one image parameter to be set manually of the corresponding group on the output unit (12) (S2) and positioning of the object on the floor mat (18) according to the at least one output imaging parameter;
c) generating an image by means of the imaging unit (06) (S3);
d) transferring the image to the computing unit (10) (S4);
e) evaluating the image in regard of at least one manually set image parameter by means of the computing unit (10) (S5), image data of the image being evaluated in the form of individual pixels and their arrangement with respect to each other for the evaluation;
f) outputting a notification by means of the output unit (12) (S5') should the evaluation yield that the at least one manually set image parameter does not correlate to the output of the image parameter to be set from method step b); and
g) repeating method steps b) to f) for each group of the picture series presetting.

2. The method according to claim 1,
**characterized in that**
method step g) also comprises saving the image in the storage unit (16).

3. The method according to claim 1 or 2,
**characterized by**
a method step f1), comprising repeating method steps b) to e) for the corresponding group of the picture series presetting should the evaluation yield that the at least one manually set image parameter does not correlate to the output of the image parameter to be set from method step b).

4. The method according to any one of the claims 1 to 3,
**characterized in that**
saving the image in a storage unit (16) comprises automatically generating a filename and/or metadata for the image.

5. The method according to any one of the claims 1 to 4,
**characterized by**
a method step b1), comprising using a setting device (22) for a stepwise manual setting of an infinitely settable image parameter of the imaging unit (06).

6. The method according to any one of the claims 1 to 5,
**characterized in that**
method step b) also comprises transferring at least one remotely settable image parameter from the databank to a unit of the imaging system (02), in particular the imaging unit (06), and correspondingly setting the image parameter on the part of the unit, in particular the imaging unit (06).

7. The method according to any one of the claims 1 to 6,
**characterized in that**
method step a) comprises selecting object data from another databank and/or inputting object data into an input unit (14) of the imaging system (02), the image parameters of the picture series presetting being adjusted in dependence of the object data.

8. The method according to any one of the preceding claims, wherein data are factored in by the imaging unit for the evaluation in method step e), which are generated by the imaging unit and are transferred by means of the imaging data, which directly or indirectly concern the imaging parameters dominant during imaging.

9. The method according to any one of the preceding claims, wherein the method comprises generating an image series as a base picture series before a medical or aesthetic procedure or treatment by executing steps a) to g) and generating a subsequent picture series after the medical or aesthetic procedure or treatment by executing steps a) to g), first method step a) being configured such when generating the base picture series that selecting the picture series presetting is saved and that the picture series is indirectly selected based on the saved picture series presetting when generating the subsequent picture series.

10. An imaging system (02) for generating a series of images of an object defined as a human or animal body, each object having predetermined image parameters, for documenting an initial state of the body before a medical or aesthetic procedure or a treatment and for document the further development of the body after the procedure or treatment, the imaging system (02) comprising a databank, an imaging unit (06), a computing unit (10), a storage unit (16) and an output unit (12), said databank comprising at least one picture series presetting comprising a plurality of predefined groups of image parameters of an object and the input unit (14) being configured for selecting a picture series presetting, the corresponding imaging parameter group having a certain part of the body to be imaged, a certain spatial orientation of the body to be imaged with respect to the imaging unit, a certain posture of the body to be imaged and/or a plurality of parameters of an imaging unit as an imaging parameter, said imaging unit (06) being configured for generating images of the object and for transferring them to the computing unit (10) and/or the storage unit (16), and said storage unit (16) being configured for storing generated images, the output unit (12) being configured for outputting at least one image parameter to be set manually of the corresponding group, and the imaging system (02) comprising a floor mat (18) for positioning the object according to the at least one output imaging parameter, the computing unit (10) being configured for evaluating an image in regard of at least one image parameter to be set manually of the group by evaluating picture data of the image in the form of individual pixels and their arrangement with respect to each other and
the computing unit (12) being configured for outputting a notification should the evaluation by means of the computing unit (10) yield that the at least one manually set image parameter does not correlate to the image parameter to be manually set on the output unit (12), the imaging system (02) further comprising a measuring device (20), which is configured for detecting the position on the floor mat (18) with respect to the imaging system, and the imaging system (02) being configured to use the determined arrangement on the floor mat (18) for adjusting the groups of imaging parameters of the picture series presetting.

11. The imaging system (02) according to claim 10,
wherein the computing unit (10) is configured for generating and for saving file-names and/or metadata in dependence of the picture series and/or the group of the picture series and/or object data and/or information input into an input unit (14) in the storage unit (16) with the images.

12. The imaging system (02) according to claim 10 or 11,
wherein the imaging system (02) comprises a setting device (22) which is arranged on the imaging unit (06) and enables a stepwise setting of an infinitely settable image parameter.

13. The imaging system (02) according to claim 12,
wherein the setting device (22) is arranged such on the imaging unit (06) that setting the focal length on a focusing optics of the imaging unit (06) in a stepwise manner is enabled using an infinitely settable focusing length.

14. The imaging system (02) according to any one of the claims 10 to 13,
wherein the imaging system (02) comprises at least one transmitting means with which at least one image parameter, which can be set remotely, is transmitted to a unit of the imaging system (02).

15. The imaging system (02) according to claim 14,
wherein the imaging system (02) comprises at least one setting means for remotely setting an image parameter, said setting means being connected to a transmitting means so as to be able to receive the corresponding remotely set image parameter.

16. The imaging system (02) according to any one of the claims 10 to 15,
wherein the computing unit (10) is configured for adjusting the image parameter to a picture series presetting using object data.

## Revendications

1. Procédé pour la génération d'une série d'images d'un objet défini comme corps humain ou animal avec des paramètres d'imagerie prédéterminés chaque fois au moyen d'un système d'imagerie (02) en partie automatisé pour la documentation d'un état initial du corps avant une procédure médicale ou esthétique ou une intervention et pour la documentation du développement ultérieur du corps après la procédure ou l'intervention, le système d'imagerie (02) comprenant une banque de données, une unité d'imagerie (06), une unité de calcul (10), une unité de stockage (16) et une unité de sortie (12), le procédé comprenant les étapes suivantes :
a) choisir un modèle de série de photos comprenant une pluralité de groupes prédéfinis de paramètres d'imagerie d'un objet de la banque de données (S1), le groupe de paramètres d'imagerie correspondant ayant une partie particulière du corps à être imagé, une orientation spatiale particulière du corps à être imagé par rapport à l'unité d'imagerie, une posture particulière du corps à être imagé et/ou une pluralité de paramètres d'une unité d'imagerie comme paramètres d'imagerie ;
― détecter l'agencement d'un essuie-pieds (18) au moyen d'un dispositif de mesure (20) du système d'imagerie (02) et utiliser l'agencement détecté de l'essuie-pieds (18) par rapport au système d'imagerie (02) pour ajuster les groupes de paramètres d'imagerie du modèle de série d'images ;
b) sortir au moins un paramètre d'imagerie à être réglé manuellement du groupe correspondant sur l'unité de sortie (12) (S2) et positionner l'objet sur l'essuie-pieds (18) selon l'au moins un paramètre d'imagerie sorti ;
c) générer une image au moyen de l'unité d'imagerie (06) (S3) ;
d) transférer l'image à l'unité de calcul (10) (S4) ;
e) évaluer l'image concernant au moins un paramètre d'imagerie réglé manuellement au moyen de l'unité de calcul (10) (S5), des données de photo de l'image étant évaluées en forme de pixels individuels et leur agencement l'un par rapport à l'autre pour l'évaluation ;
f) sortir une notification au moyen de l'unité de sortie (12) (S5') si l'évaluation montre que l'au moins un paramètre d'imagerie réglé manuellement ne correspond pas à la sortie du paramètre d'imagerie à être réglé dans l'étape de procédé b) ; et
g) répéter les étapes de procédé b) à f) pour chaque groupe du modèle de série de photos.

2. Procédé selon la revendication 1,
**caractérisé en ce que**
l'étape de procédé g) comprend aussi l'étape consistant à stocker l'image dans l'unité de stockage (16).

3. Procédé selon la revendication 1 ou 2,
**caractérisé par**
une étape de procédé f1), comprenant l'étape consistant à répéter les étapes de procédé b) à e) pour le groupe correspondant du modèle de série de photos si l'évaluation montre que l'au moins un paramètre d'imagerie réglé manuellement ne correspond pas à la sortie du paramètre d'imagerie à être réglé dans l'étape de procédé b).

4. Procédé selon l'une quelconque des revendications 1 à 3,
**caractérisé en ce que**
le stockage de l'image dans une unité de stockage (16) comprend la génération automatique d'un nom de fichier et/ou de métadonnées de l'image.

5. Procédé selon l'une quelconque des revendications 1 à 4,
**caractérisé par**
une étape de procédé b1), comprenant l'utilisation d'un dispositif de réglage (22) pour un réglage manuel graduel d'un paramètre d'imagerie réglable en continu de l'unité d'imagerie (06).

6. Procédé selon l'une quelconque des revendications 1 à 5,
**caractérisé en ce que**
l'étape de procédé b) comprend aussi le transfert d'au moins un paramètre d'imagerie réglable de manière télécommandée de la banque de données à une unité du système d'imagerie (02), notamment l'unité d'imagerie (06), et le réglage correspondant du paramètre d'imagerie de la part de l'unité, notamment de l'unité d'imagerie (06).

7. Procédé selon l'une quelconque des revendications 1 à 6,
**caractérisé en ce que**
l'étape de procédé a) comprend la sélection de données d'objet d'une autre banque de données et/ou l'entrée de données d'objet sur une unité d'entrée (14) du système d'imagerie (02), les paramètres d'imagerie du modèle de série de photos étant ajustés en fonction des données d'objet.

8. Procédé selon l'une quelconque des revendications précédentes, dans lequel des données qui sont générées par l'unité d'imagerie et qui sont transférées avec les données d'imagerie qui concernent directement ou indirectement les paramètres d'imagerie dominants pendant l'imagerie sont aussi prises en compte par l'unité d'imagerie pour l'évaluation dans l'étape de procédé e).

9. Procédé selon l'une quelconque des revendications précédentes, dans lequel le procédé comprend la génération d'une série d'images comme série de photos de base avant une procédure médicale ou esthétique ou une intervention par exécuter les étapes a) à g) et la génération d'une série de photos suivante après la procédure médicale ou esthétique ou l'intervention par exécuter les étapes a) à g), la première étape de procédé a) étant configurée de telle manière quand la série de photos de base est générée que la sélection du modèle de série de photos est stockée et que la série de photos est choisie indirectement basée sur le modèle de série de photos stocké quand la série de photos suivante est générée.

10. Système d'imagerie (02) pour la génération d'une série d'images d'un objet défini comme corps humain ou animal avec des paramètres d'imagerie prédéterminés chaque fois pour la documentation d'un état initial du corps avant une procédure médicale ou esthétique ou une intervention et pour la documentation du développement ultérieur du corps après la procédure ou l'intervention, le système d'imagerie (02) comprenant une banque de données, une unité d'imagerie (06), une unité de calcul (10), une unité de stockage (16) et une unité de sortie (12), la banque de données comprenant au moins un modèle de série de photos comprenant une pluralité de groupes prédéfinis de paramètres d'imagerie d'un objet et l'unité d'entrée (14) étant configurée pour la sélection d'un modèle de série de photos, le groupe de paramètres d'imagerie correspondant ayant une partie particulière du corps à être imagé, une orientation spatiale particulière du corps à être imagé par rapport à l'unité d'imagerie, une posture particulière du corps à être imagé et/ou une pluralité de paramètres d'une unité d'imagerie comme paramètres d'imagerie, l'unité d'imagerie (06) étant configurée pour générer des images de l'objet et pour les transférer à l'unité de calcul (10) et/ou l'unité de stockage (16), et l'unité de stockage (16) étant configurée pour stocker des images générées, l'unité de sortie (12) étant configurée pour sortir au moins un paramètre d'imagerie à être réglé manuellement du groupe correspondant, et le système d'imagerie (02) comprenant un essuie-pieds (18) pour positionner l'objet selon l'au moins un paramètre d'imagerie sorti, l'unité de calcul (10) étant configurée pour évaluer une image concernant au moins un paramètre d'imagerie à être réglé manuellement du groupe en évaluant des données de photo de l'image en forme de pixels individuels et leur agencement l'un par rapport à l'autre et
l'unité de calcul (12) étant configurée pour sortir une notification si l'évaluation au moyen de l'unité de calcul (10) montre que l'au moins un paramètre d'imagerie réglé manuellement ne correspond pas à la sortie du paramètre d'imagerie à être réglé manuellement sur l'unité de sortie (12),
le système d'imagerie (02) comprenant en outre un dispositif de mesure (20), qui est configuré pour détecter la position de l'essuie-pieds (18) par rapport au système d'imagerie, et le système d'imagerie (02) étant configuré pour utiliser l'agencement détecté de l'essuie-pieds (18) par rapport au système d'imagerie (02) pour ajuster les groupes de paramètres d'imagerie du modèle de série de photos.

11. Système d'imagerie (02) selon la revendication 10,
dans lequel l'unité de calcul (10) est configurée pour générer des noms de fichier et/ou des métadonnées en fonction de la série de photos et/ou du groupe de la série de photos et/ou de données d'objet et/ou d'informations entrées sur une unité d'entrée (14) et pour les stocker dans l'unité de stockage (16) avec les images.

12. Système d'imagerie (02) selon la revendication 10 ou 11,
dans lequel le système d'imagerie (02) comprend un dispositif de réglage (22) qui est disposé sur l'unité d'imagerie (06) et qui permet un réglage graduel d'un paramètre d'imagerie réglable en continu.

13. Système d'imagerie (02) selon la revendication 12,
dans lequel le dispositif de réglage (22) est disposé de telle manière sur l'unité d'imagerie (06) qu'un réglage graduel de la focale sur une optique d'imagerie de l'unité d'imagerie (06) ayant une focale réglable en continu est permis.

14. Système d'imagerie (02) selon l'une quelconque des revendications 10 à 13, dans lequel le système d'imagerie (02) comprend au moins un moyen de transfert au moyen duquel au moins un paramètre d'imagerie qui peut être réglé de manière télécommandé est transmis à une unité du système d'imagerie (02).

15. Système d'imagerie (02) selon la revendication 14,
dans lequel le système d'imagerie (02) comprend au moins un moyen de réglage pour le réglage télécommandé d'un paramètre d'imagerie, le moyen de réglage étant relié à un moyen de transfert afin de recevoir le paramètre d'imagerie réglable de manière télécommandée correspondant.

16. Système d'imagerie (02) selon l'une quelconque des revendications 10 à 15, dans lequel l'unité de calcul (10) est configurée pour ajuster les paramètres d'imagerie d'un modèle de série de photos en utilisant des données d'objet.
